(19) Europäisches Patentamt
European Patent Office
Office européen des brevets

(11) Veröffentlichungsnummer : **0 003 765**
**B1**

(12) # EUROPÄISCHE PATENTSCHRIFT

(45) Veröffentlichungstag der Patentschrift : **11.02.81**

(51) Int. Cl.³ : **C 07 D251/34**, C 08 G 18/75, C 08 G 18/80, C 09 D 3/72

(21) Anmeldenummer : **79100339.5**

(22) Anmeldetag : **06.02.79**

(54) **Verfahren zur Herstellung von Isocyanuratgruppen aufweisenden Polyisocyanaten auf Basis von 1-Isocyanato-3,3,5-trimethyl-5-isocyanatomethylcyclohexan und ihre Verwendung als Isocyanatkomponente in Polyurethanlacken.**

(30) Priorität : **17.02.78 DE 2806731**

(43) Veröffentlichungstag der Anmeldung : **05.09.79 (Patentblatt 79/18)**

(45) Bekanntmachung des Hinweises auf die Patenterteilung : **11.02.81 Patentblatt 81/06**

(84) Benannte Vertragsstaaten : **BE CH DE FR GB IT NL SE**

(56) Entgegenhaltungen :
**DE - A - 1 667 309**
**DE - A - 2 325 826**
**DE - A - 2 551 634**
**DE - A - 2 644 684**
**DE - C - 1 150 080**
**GB - A - 1 386 399**
**GB - A - 1 391 066**

(73) Patentinhaber : **BAYER AG**
**Zentralbereich Patente, Marken und Lizenzen**
**D-5090 Leverkusen 1, Bayerwerk (DE)**

(72) Erfinder : **Bock, Manfred, Dr.**
**Haydnstrasse 18**
**D-5090 Leverkusen (DE)**
Erfinder : **Pedain, Josef, Dr.**
**Haferkamp 6**
**D-5000 Köln 80 (DE)**
Erfinder : **Slawyk, Wilhelm, Dr.**
**Walter-Flex-Strasse 25**
**D-5090 Leverkusen 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

**EP 0 003 765 B1**

Verfahren zur Herstellung von Isocyanuratgruppen aufweisenden Polyisocyanaten auf Basis von 1-Isocyanato-3,3,5-trimethyl-5-isocyantomethylcyclohexan und ihre Verwendung als Isocyanatkomponente in Polyurethanlacken

Die vorliegende Erfindung betrifft ein neues Verfahren zur Herstellung von Isocyanuratgruppen aufweisenden Polyisocyanaten durch teilweise Trimerisierung der Isocyanatgruppen von 1-Isocyanato-3,3,5-trimethyl-5-isocyanatomethyl-cyclohexan (= Isophorondiisocyanat, abgekürzt : IPDI).

Die Cyclopolymerisation von IPDI ist bereits bekannt. Gemäß DE-OS 1 934 763 entsteht bei Verwendung von Phosphinen als Katalysator ein Reaktionsgemisch, welches neben dem Dimerisierungsprodukt auch Isocyanuratgruppen aufweisende Polyisocyanate enthält.

Diese Seite ist ersatzlos zu streichen.

Ein technisch brauchbares, einfaches Verfahren zur Herstellung von Isocyanuratgruppen aufweisenden Polyisocyanaten auf Basis von IPDI steht nicht zur Verfügung. Da jedoch andererseits ein weitgehend farbloses, lösungsmittel- und weitgehend monomerenfreies, Isocyanuratgruppen aufweisendes Polyisocyanat auf Basis von IPDI ein äußerst interessantes Lackpolyisocyanat darstellen würde, war es die Aufgabe der vorliegenden Erfindung ein derartiges Verfahren zur Verfügung zu stellen.

Diese Aufgabe konnte überraschenderweise durch Verwendung ganz bestimmter quaternärer Ammoniumhydroxide als Katalysator zur Trimerisierung von IPDI gelöst werden.

Die Verwendung von quaternären Ammoniumhydroxiden als Trimerisierungskatalysator für Isocyanate ist aus der DE-AS 1 150 080 bereits bekannt. Obwohl aus dieser Vorveröffentlichung die Abstoppung der Polymerisation durch thermische Zersetzung des Katalysators und auch die Möglichkeit eines erneuten Zusatzes einer Katalysatormenge (die sogenannte Nachkatalyse) bekannt ist, eignen sich die in dieser Literaturstelle genannten Ammoniumhydroxide kaum als technisch brauchbare Katalysatoren insbesondere zur lösungsmittelfreien Trimerisierung von IPDI, da in Abwesenheit von Lösungsmitteln die Reaktion nicht kontrollierbar ist. Insbesondere muß beim Versuch einer Trimerisierung von aliphatischen bzw. cycloaliphatischen Diisocyanaten mit den Ammoniumhydroxiden der genannten Veröffentlichung oft ein plötzlicher stark exothermer Reaktionsbeginn nach einer mehr oder weniger langen Inkubationszeit beobachtet werden. In den Ausführungsbeispielen der DE-AS 1 150 080 wird demzufolge im wesentlichen auch nur die Trimerisierung von aromatischen Isocyanaten beschrieben. Als einziges Beispiel der Trimerisierung eines aliphatischen Isocyanats wird in Beispiel 13 die Trimerisierung von Hexadecylisocyanat erwähnt. Nach 4-tägiger Umsetzung wird in weniger als 50 % Ausbeute das entsprechende Trimerisat erhalten. Für eine wirtschaftliche, lösungsmittelfreie Trimerisierung von IPDI in einer reproduzierbaren und leicht kontrollierbaren Reaktion sind die Katalysatoren der DE-AS 1 150 080 ungeeignet.

Auch die in DE-OS 2 631 733 bzw. in GB-PS 1 465 812 genannten quaternären Ammoniumsalze, -alkoholate bzw. -phenolate sind für eine derartige Umsetzung als Katalysatoren wenig geeignet, da die Verbindungen thermisch nicht leicht zerstörbar sind und daher beim gewünschten Trimerisierungsgrad durch Zugabe eines Katalysatorengifts desaktiviert werden müssen, was wiederum zu unerwünschten Verunreinigungen im Verfahrensprodukt führt.

Demgegenüber gestatten die erfindungsgemäß einzusetzenden Katalysatoren eine lösungsmittelfreie Herstellung von Isocyanuratgruppen aufweisenden Polyisocyanaten auf Basis von IPDI, wobei eine Desaktivierung des Katalysators beispielsweise durch Zugabe eines Katalysatorengifts unterbleiben kann, da die erfindungsgemäß als Katalysatoren einzusetzenden Verbindungen sich während der Umsetzung thermisch zersetzen und dadurch ihre Aktivität verlieren.

Aus der DE-OS 1 667 309 sind zwar schon Mehrkomponenten-Gemische bekannt, die als Trimerisierungskatalysatoren für Isocyanate geeignet sind, und in denen quaternäre Ammoniumhydroxide in Kombination mit einem Phenol, einem Oxim oder Methanol vorliegen, jedoch wird in dieser Vorveröffentlichung neben zahlreichen quaternären Ammoniumhydroxiden der auch in DE-AS 1 150 080 genannten Art Trimethyl-hydroxyäthyl-ammonium-hydroxid als einziges Hydroxyalkyl-ammonium-hydroxid nur beiläufig genannt. In den Beispielen wird jedoch nur die Verwendung von hydroxylsubstituentenfreien quaternären Hydroxiden zur Trimerisierung von aromatischen Polyisocyanaten bzw. von n-Hexylisocyanat erläutert. Irgendein richtungsweisender Hinweis, Isophorondiisocyanat unter Verwendung von Trimethylhydroxyäthylammoniumhydroxid zu trimerisieren, kann der Vorveröffentlichung nicht entnommen werden.

Gegenstand der vorliegenden Erfindung ist ein Verfahren zur Herstellung von Isocyanuratgruppen aufweisenden Polyisocyanaten durch teilweise Trimerisierung der Isocyanatgruppen von 1-Isocyanato-3,3,5-trimethyl-5-isocyanatomethyl-cyclohexan unter Verwendung von die Trimerisierung von Isocyanatgruppen beschleunigenden Katalysatoren, dadurch gekennzeichnet, daß man als Katalysator mindestens eine Hydroxylgruppe aufweisende, quaternäre Hydroxyalkylammonium-hydroxide der formel

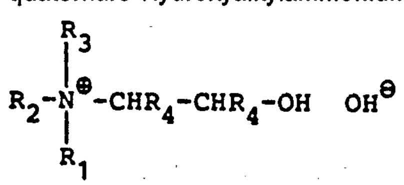

in welcher

$R_1$, $R_2$ und $R_3$ für gleiche oder verschiedene Reste stehen und gegebenenfalls Hydroxyl-substituierte Alkylgruppen mit 1 bis 20 Kohlenstoffatomen, gegebenenfalls mit Hydroxylgruppen substituierte Cycloalkylreste mit 4 bis 15 Kohlenstoffatomen, gegebenenfalls mit Hydroxylgruppen substituierte Aralkylreste mit 7 bis 15 Kohlenstoffatomen oder gegebenenfalls mit Hydroxylgruppen substituierte Arylreste mit 6 bis 15 Kohlenstoffatomen bedeuten, wobei zwei der genannten Reste $R_1$, $R_2$ oder $R_3$ auch zusammen mit dem Stickstoffatom und gegebenenfalls zusammen mit einem Sauerstoff- oder einem weiteren Stickstoff-Heteroatom einen heterocyclischen Ring mit 4-6 Kohlenstoffatomen bilden können, oder wobei die Reste $R_1$, $R_2$ und $R_3$ jeweils für Äthylenreste stehen die zusammen mit dem quartären Stickstoffatom und einem weiteren tertiären Stickstoffatom ein bicyclisches Triäthylen-diamin-Gerüst bilden,

$R_4$ für Wasserstoff und/oder eine Alkylgruppe mit 1 bis 12 Kohlenstoffatomen, eine Cycloalkylgruppe mit 5 bis 7 Kohlenstoffatomen, eine Aralkylgruppe mit 7 bis 10 Kohlenstoffatomen, eine Arylgruppe mit 6 bis 10 Kohlenstoffatomen oder eine $R_5$-O-$(CH_2)_n$-Gruppe darstellt, in welcher $R_5$ für Wasserstoff, einen Alkylrest mit 1 bis 12 Kohlenstoffatomen, einen Cycloalkylrest mit 4 bis 10 Kohlenstoffatomen, einen Aralkylrest mit 7 bis 10 Kohlenstoffatomen oder einen Arylrest mit 6 bis 10 Kohlenstoffatomen steht und n eine ganze zahl von 1 bis 6 bedeutet, verwendet.

Gegenstand der vorliegenden Erfindung ist auch die Verwendung der erfindungsgemäßen Verfahrensprodukte, gegebenenfalls in mit Blockierungsmitteln für Isocyanatgruppen blockierter Form, als Isocyanatkomponente in Polyurethanlacken.

Zu den bevorzugten erfindungsgemäß einzusetzenden Katalysatoren gehören Verbindungen der letztgenannten Formel, bei welchen

$R_1$, $R_2$ und $R_3$ für gleiche oder verschiedene Alkylreste mit 1 bis 4 Kohlenstoffatomen stehen und $R_4$ Wasserstoff bedeutet.

Ein besonders bevorzugter Katalysator ist N,N,N-Trimethyl-N-(2-hydroxyäthyl)-ammonium-hydroxid.

Die für das erfindungsgemäße Verfahren als Katalysatoren geeigneten Verbindungen werden hergestellt aus tert. Aminen wie beispielsweise Trimethylamin, Tributylamin, 2-Dimethylaminoäthanol, Triäthanolamin, Dodecyldimethylamin, N,N-Dimethylcyclohexylamin, N-Methylpyrrolidin, N-Methylmorpholin oder 1,4-Diazabicyclo-2,2,2-octan und Alkylenoxyden wie Äthylenoxid, Propylenoxid, Butylenoxid-1,2, Styroloxid, Methoxy-, Äthoxy- oder Phenoxypropylenoxid.

Die Darstellung der Katalysatoren erfolgt in an sich bekannter Weise durch Umsetzung von Alkylenoxid und tert. Amin in wäßrig-alkoholischem Medium (vgl. US-PS 3 995 997, Kol. 2, Zeilen 19-44). Als Alkohole eignen sich beispielsweise Methanol, Äthanol, Propanol oder tert.-Butanol. Destillierbare Anteile, wie das Wasser oder das an der Umsetzung mit Alkylenoxid nicht teilnehmende tert.-Butanol, werden anschließend entfernt. Der Gehalt an quartärer Base wird auf analytischem Wege, beispielsweise titrimetrisch ermittelt, so daß die für die Trimerisierung jeweils benötigte Menge bestimmt werden kann.

Die Katalysatoren werden im allgemeinen in einer Menge von 0,01 bis 1 Gew.-%, bevorzugt von 0,03 bis 0,3 Gew.-% bezogen auf eingesetztes IPDI angewendet. Sie können in reiner Form oder als Lösung eingesetzt werden. Als Lösungsmittel eignen sich je nach Art des Katalysators beispielsweise Toluol, Dimethylformamid, Dimethylsulfoxid oder auch Mischungen dieser Lösungsmittel. Bei der Mitverwendung von Carbamidsäurederivate bildenden Hydroxyverbindungen als Cokatalysatoren (s.u.) ist es von Vorteil, diese als Lösungsmittel der Katalysatoren zu verwenden. Als solche kommen beispielsweise in Frage Methanol, Äthanol, 2-Äthyl-hexanol oder Glykole wie Äthandiol, Butandiol oder 2-Äthyl-hexandiol.

Das erfindungsgemäße Verfahren weist verschiedene, grundlegende Vorteile auf : Die Trimerisierung verläuft bei entsprechender Dosierung des Katalysators langsam und stetig (ohne Inkubationszeit) ab. Hierdurch kann lösungsmittelfrei gearbeitet werden. Eine nicht genügend fortgeschrittene Trimerisierung läßt sich nachkatalysieren bis der gewünschte Trimerisierungsgrad erreicht ist, was nach den Trimerisierungsverfahren des Standes der Technik nicht ohne weiteres möglich ist. Aufgrund der Thermoinstabilität der Katalysatoren ist eine Beendigung der Trimerisierung durch Abstopper nicht erforderlich. Störende Trübungen durch Salzbildung mit Abstoppern treten dementsprechend bei den Trimerisatprodukten auch in großer Verdünnung nicht auf. Ein weiterer Vorteil der Thermoinstabilität der erfindungsgemäß einzusetzenden Katalysatoren ist in dem Umstand zu sehen, daß ein unkontrolliertes Durchpolymerisieren des Reaktionsansatzes praktisch ausgeschlossen ist, da durch die in einem solchen Falle zunächst eintretende starke Temperaturerhöhung automatisch die Zersetzungstemperatur des eingesetzten Katalysators erreicht und damit eine Abbruch der Reaktion bewirkt würde.

Die Mitverwendung von Cokatalysatoren ist beim erfindungsgemäßen Verfahren möglich jedoch nicht erforderlich. Als Cokatalysatoren kommen Substanzen in Frage von denen bekannt ist, daß sie Isocyanate polymerisieren. Sie werden in Megen von 1-90, vorzugsweise 1-50 Gew.-% des verwendeten Katalysators eingesetzt. Als Cokatalysatoren kommen z. B. folgende Substanzen in Frage : tert. Amine, wie Triäthylamin, Tributylamin, N-Methyl-morpholin, N-Äthyl-morpholin, N-Cocomorpholin, N,N,N',N'-Tetramethyläthylendiamin, 1,4-Diaza-bicyclo-(2,2,2)-octan, N-Methyl-N'-dimethyl-aminoäthyl-piperazin, N,N-Dimethylbenzylamin, Bis-(N,N-diäthylaminoäthyl)-adipat, N,N-Diäthylbenzylamin, Pentamethyldiäthylentriamin, N,N-Dimethylcyclohexylamin, N,N,N',N'-Tetramethyl-1,3-butandiamin, N,N-Dimethyl-β-phenyläthyl-amin, 1,2-Dimethylimidazol, 2-Methylimidazol.

Als Cokatakysatoren eignen sich ebenfalls Mannichbasen aus sekundären Aminen, wie Dimethylamin, Diäthylamin oder Morpholin und Aldehyden, vorzugsweise Formaldehyd, oder Ketonen wis Aceton,

Methyläthylketon oder Cyclohexanon und Phenolen, wie Phenol, Nonylphenol oder Bisphenol. Als Zusatzkatalysatoren eignen sich auch einige gegenüber Isocyanatgruppen aktive Wasserstoffatome aufweisende tert. Amine, wie beispielsweise Triäthanolamin, Triisopropanolamin, N-Methyldiäthanolamin, N,N-Dimethyläthanolamin, sowie deren Umsetzungsprodukte mit Alkylenoxiden, wie Propylenoxid und/oder Äthylenoxid. Als Cokatalysatoren kommen ferner aliphatische, araliphatische und gemischt aliphatisch-aromatische Phosphine in Frage, z.B. Triäthylphosphin, Tri-n-butylphosphin, Dimethylbenzylphosphin, Dimethylphenylphosphin, Tribenzylphosphin oder P-Butyl-phosphacyclopentan.

Weiter eignen sich Silaamine mit Kohlenstoff-Silicium-Bindungen, wie sie z. B. in der deutschen Patentschrift 1 229 290 beschrieben sind, z.B. 2,2,4-Trimethyl-2-silamorpholin oder 1,3-Diäthylaminomethyl-tetramethyl-disiloxan.

Die Mitverwendung von Carbamidsäureestern analog GB-PS 949 253 oder DE-AS 1 013 869 ist ebenfalls möglich. Die mitzuverwendenden Carbamidsäureester werden durch Umsetzung von aliphatischen, araliphatischen oder aromatischen Mono- oder Polyhydroxylverbindungen mit Mono- oder Polyisocyanaten, zweckmäß gerweise mit dem hier verwendeten IPDI hergestellt. Es ist ohne Einfluß auf den Polymerisationsverlauf, ob ein vorgebildeter und isolierter Carbamidsäureester dem zu polymerisierenden IPDI zugesetzt wird oder ob dieser erst während der Trimerisierungsreaktion gebildet wird, wenn man beispielsweise die Hydroxyverbindung zusammen mit dem Katalysator, etwa als Lösung, in das Isocyanat einbringt. Geeignete Hydroxyverbindungen, die mit IPDI zu den als Co-Katalysatoren wirksamen Carbamidsäurederivaten reagieren und gleichzeitig Löser der eigentlich als Trimerisierungskatalysator wirksamen quartären Stickstoffbasen darstellen, sind beispielsweise Methanol, Äthanol, 2-Äthylhexanol oder Glykole, wie Äthandiol, Butandiol oder 2-Äthylhexandiol. Neben diesen Cokatalysatoren lassen sich auch weitere basisch reagierende Substanzen mitverwenden, wie Alkali- und Erdalkalihydroxide, Alkalialkoholate und -Phenolate, sowie Alkali- und Erdalkalisalze von Carbon- bzw. höheren Fettsäuren.

Selbstverständlich ist es auch möglich Mischungen verschiedener Cokatalysatoren einzusetzen oder andere Katalysatorsysteme mitzuverwenden, die in der Lage sind, die Isocyanatreaktionen zu beschleunigen, wie metallorganische Verbindungen von Zinn, Antimon oder Blei. Vorzugsweise werden jedoch solche verwendet, die bei der Trimerisation eingebaut werden, wie Carbamidsäurederivate bildende Hydroxyverbindungen oder solche, die ebenfalls, wie die verwandten Trimerisierungskatalysatoren, thermisch desaktivierbar sind, so z.B. die Mannichbasen.

Als Ausgangsmaterial für das erfindungsgemäße Verfahren dient vorzugsweise technisch reines, d.h. destilliertes und farbloses IPDI.

Die erfindungsgemäße Umsetzung erfolgt bei 30-90 °C vorzugsweise 40-80 °C. Die Umsetzung wird im allgemeinen, von den geringen Lösungsmittelmengen für den Katalysator abgesehen, lösungsmittelfrei durchgeführt, obwohl selbstverständlich die Mitverwendung der an sich bekannten Lacklösungsmittel prinzipiell nicht ausgeschlossen ist.

Es ist einer der wesentlichen Vorteile der erfindungsgemäß einzusetzenden Katalysatoren, daß sie ihre Wirksamkeit sofort, d.h. ohne Inkubationszeit entfalten. Gleichzeitig beginnen sich die erfindungsgemäß einzusetzenden Katalysatoren in den angegebenen Temperaturbereichen thermisch zu zersetzen, so daß der Trimerisationsgrad (Prozentsatz der trimerisierten Isocyanatgruppen bezogen auf Gesamtmenge der ursprünglich vorliegenden Isocyanatgruppen) bei vorgewählter Ausgangstemperatur auf einfache Weise durch die Menge an zugesetztem Katalysator gesteuert werden kann. Gewünschtenfalls kann auch der Trimerisationsgrad durch Zugabe einer weiteren Katalysatorenmenge nach thermischer Zersetzung einer ersten Katalyzatorenmenge weiter erhöht werden.

Im allgemeinen wird die Katalysatorenmenge so bemessen, daß das erhaltene Reaktionsgemisch (ohne ggf. mitverwendete Lösungsmittel) ein NCO-Gehalt von ca. 25 bis 35 vorzugsweise 27 bis 32 Gew.-% aufweist. Nicht umgesetztes, überschüssiges IPDI kann anschließend in an sich bekannter Weise beispielsweise durch Dünnschichtdestillation entfernt werden.

Das erfindungsgemäße Verfahren kann beispielsweise wie folgt durchgeführt werden :

IPDI wird in einem Dreihalskolben unter Stickstoff (die Mitverwendung von Inertgas ist nicht unbedingt erforderlich) vorgelegt und auf eine Temperatur in Bereich von 40 bis 70 °C, beispielsweise 50 °C erwärmt. In diese Vorlage trägt man die Katalysatorlösung ein. Die Trimerisierung beginnt augenblicklich nachdem die Katalysatorlösung im IPDI eingerührt ist. Die Temperatur steigt langsam innerhalb von 30 bis 60 Minuten auf 70-80 °C. Bei dieser Temperatur wird eine Stunde nachgerührt, wobei der Katalysator auf Grund seiner Thermoinstabilität desaktiviert wird. Hiernach wird die Trimerisatlösung im Hochvakuum von überschüssigem IPDI befreit (Dünnschichtdestillation). In Abhängigkeit vom Trimerisationsgrad erhält man hierbei als Destillationsrückstand das erfindungsgemäße Verfahrensprodukt als hellgelbes, sprödes Harz, welches 75 Gew.-% ig in Äthylglykolacetat gelöst bei 25 °C eine Viskosität von maximal 50 000 mPas, vorzugsweise von unter 20 000 mPas, einen NCO-Gehalt von ca. 9,5 bis 15,5 Gew.-%, vorzugsweise von ca. 11 bis 14 Gew.-% und einen Gehalt an freiem IPDI von unter 3 Gew.-%, vorzugsweise unter 1 Gew.-% aufweist.

Ausschlaggebend für die Qualität des Endproduktes (Viskosität der Harzlösung), ist der schonende Verlauf der Trimerisierung. Die Katalysatormenge ist deshalb so zu bemessen, daß kein zu schneller Reaktionsverlauf erfolgt. Zweckmäßig ist es deshalb auch, in zwei oder mehreren Etappen die Dosierung der Katalysatorlösung vorzunehmen. Hierbei wird zunächst, wie oben dargelegt, das IPDI auf eine

geeignete Ausgangstemperatur, wie beispielsweise 60 °C, erwärmt. In diese Vorlage läßt man nun beispielsweise 1/2 bis 2/3 der Gesamtmenge an Katalysatorlösung einlaufen. Die Temperatur steigt mit beginnender Trimerisation und erreicht nach 15 bis 30 Minuten ihr erstes Maximum. Bei dieser Temperatur läßt man nachrühren, bis die Reaktion abklingt und gibt zu diesem Zeitpunkt einen weiteren Teil der Katalysatorlösung hinzu. Hierdurch steigt die Temperatur erneut an und erreicht nach ca. 15 Minuten 80 °C. Nach einigem Nachrühren stellt sich ein konstanter NCO-Wert ein, der selbst bei längerem Nachrühren nicht mehr wesentlich sinkt. Ist der gewünschte NCO-Gehalt der Trimerisatlösung zu diesem Zeitpunkt noch nicht vollständig erreicht, läßt sich durch weitere Katalysatorzugabe bei 80 °C der Endpunkt einstellen. Diese Nachkatalyse erfolgt im Temperaturbereich zwischen 80 und 85, maximal 90 °C. Als besonders günstig für die Qualität der Verfahrensprodukte hat sich eine Trimerisation bis zu einem NCO-Gehalt der noch überschüssiges IPDI enthaltenden Reaktionsmischung von 30 ± 1 Gew.-% erwiesen, der sich nach der hier beschriebenen Methode gut einstellen läßt. Sobald dieser NCO-Gehalt erreicht ist wird 1/2 Stunde nachgerührt und hiernach das überschüssige IPDI im Hochvakuum abdestilliert. Ebenfalls von Vorteil im Sinne einer langsam fortschreitenden Trimerisierung ist das stetige Eindosieren der Katalysatorlösung in die IPDI-Vorlage bis zum angestrebten NCO-Wert. Diese Methode ist besonders bei größeren Ansätzen geeignet, bei denen mit Hilfe von Dosierpumpen in hohem Maße ein gleichmäßiges Eintragen möglich ist.

Bei einer weiteren Verfahrensvariante wird das bei Raumtemperatur inaktive Verhalten der Katalysatorlösung gegenüber IPDI ausgenutzt. Eine Mischung von IPDI und Katalysatorlösung in einem für die Trimerisierung geeigneten Verhältnis ist bei 20-25 °C über einen längeren Zeitraum stabil, erst bei erhöhten Temperaturen erfolgt die Trimerisierung. Hierzu wird die genannte Mischung entweder über eine Förderpumpe oder bei kleineren Ansätzen mit Hilfe eines Tropftrichters in eine auf Temperaturen zwischen 60 und 90 °C, beispielsweise 75 °C, erwärmte Vorlage eingetragen. Sehr rasch stellt sich ein über den Eindosierzeitraum nahezu konstanter, durch das Verhältnis von Katalysator zu IPDI bestimmter NCO-Gehalt der Trimerisatlösung ein. Durch Variation von Katalysatorkonzentration lassen sich bestimmte NCO-Werte gezielt einstellen. Diese sehr schonende Verfahrensweise führt zu farbhellen Trimerisaten mit niedriger Viskosität der 75 % igen Lösung in Äthylglykolacetat.

Bei der Mitverwendung eines Cokatalysators wird dieser vor der Trimerisierungsreaktion in das IPDI eingerührt oder mit dem Trimerisierungskatalysator zusammen eingegeben. Die Polymerisationsreaktion kann auch in diesem Fall nach den obengenannten Alternativen geführt werden und zwar durch eine einmalige, portionsweise oder kontinuierliche Zugabe der entsprechend bemessenen Katalysatorlösung. Ein Abstoppen der Reaktion mit den üblichen, hierzu brauchbaren Alkylierungs-Substanzen, ist wegen der thermischen Desaktivierbarkeit der verwendeten Katalysatoren, wie bereits hervorgehoben, nicht erforderlich, jedoch nicht ausgeschlossen. Naturgemäß ist sie dann notwendig, wenn ein Cokatalysator mitverwendet wird, der nicht thermisch desaktivierbar ist.

Die Trimerisation des IPDI kann mit den gleichen Katalysatoren und nach einem ähnlichen Reaktionsablauf auch kontinuierlich in einer Kesselkaskade durchgeführt werden. Von Vorteil ist hierbei ebenfalls die Möglichkeit einer thermischen Beendigung der Trimerisation.

Das erfindungsgemäße Verfahren weist gegenüber den Verfahren des Standes der Technik wesentliche Vorteile auf:

1. Das Verfahren ist in jeder Phase leicht kontrollierbar, da die erfindungsgemäß einzusetzenden Katalysatoren einerseits ohne Inkubationszeit ihre Aktivität entfalten, und da sie thermisch labile Substanzen darstellen, die sich bei den Umsetzungstemperaturen thermisch zersetzen, so daß eine Desaktivierung durch Zugabe eines Katalysatorengifts unterbleiben kann.

2. Ein Einstellen auf bestimmte gewünschte Endwerte der Trimerisation ist beim erfindungsgemäßen Verfahren durch Nachkatalyse möglich, so daß Produkte von gleichbleibender Qualität hergestellt werden können.

3. Das Verfahren ist technisch einfach durchführbar und preisgünstig.

4. Das Verfahren gestattet die Herstellung von in gebräuchlichen Lacklösungsmitteln leicht löslichen, von überschüssigem IPDI leicht zu befreienden, physiologisch weitgehend unbedenklichen modifizierten Polyisocyanaten einer hellen Eigenfarbe.

Die erfindungsgemäßen Verfahrensprodukte stellen wertvolle Ausgangsmaterialien zur Herstellung von Polyurethankunststoffen nach dem Isocyanat-Polyadditionsverfahren, insbesondere zur Herstellung von Ein- oder Zweikomponenten-Polyurethanlacken dar.

Die erfindungsgemäßen Verfahrensprodukte stellen in mit den an sich bekannten Blockierungsmitteln blockierter Form wertvolle Ausgangsmaterialien für Zweikomponenten-Polyurethan-Einbrennlacke dar.

Bevorzugte Reaktionspartner für die erfindungsgemäßen, gegebenenfalls in blockierter Form vorliegenden Verfahrensprodukte bei der Herstellung von Polyurethanlacken sind die in der Polyurethanlacktechnik an sich bekannten Polyhydroxypolyester (wobei insbesondere Alkydharze in Frage kommen), Polyhydroxypolyacrylate und gegebenenfalls niedermolekularen, mehrwertigen Alkohole. Auch Polyamine, insbesondere in blockierter Form als Polyketimine oder Oxazolidine sind denkbare Reaktionspartner für die erfindungsgemäßen Verfahrensprodukte. Die Mengenverhältnisse, in welchen die erfindungsgemäßen, gegebenenfalls blockierten Polyisocyanate und die genannten Reaktionspartner bei der Herstellung von Polyurethanlacken umgesetzt werden, werden im allgemeinen so gewählt, daß auf eine

(gegebenenfalls blockierte) Isocyanatgruppe 0,8-3, vorzugsweise 0,9-1, 1, Hydroxyl-, Amino-, und/oder Carboxylgruppen entfallen.

Zur Beschleunigung der Aushärtung können in bekannter Weise die in der Isocyanatchemie üblichen Katalysatoren verwendet werden, wie z.B. tert. Amine wie Triäthylamin, Pyridin, Methylpyridin, Benzyldimethylamin, N,N-Dimethylaminocyclohexan, N-Methylpiperidin, Pentamethyldiäthylentriamin, N,N'-Endoäthylenpiperazin, N,N'-Dimethylpiperazin usw., Metallsalze wie Eisen(III)-chlorid, Zinkchlorid, Zink-2-äthylcaproat, Zinn(II)-2-äthylcaproat, Dibutylzinn-(IV)-dilaurat, Molybdänglykolat usw.

Bei Verwendung der erfindungsgemäßen Verfahrensprodukte in Einbrennlacken werden die NCO-Gruppen ganz oder teilweise in bekannter Weise blockiert. Das Polyisocyanat wird mit einem geeigneten Blockierungsmittel, vorzugsweise bei erhöhter Temperatur (z.B. 40 bis 140 °C) gegebenenfalls in Gegenwart eines geeigneten Katalysators, wie z.B. tert.-Amine, Metallsalze wie Zink-2-äthylcaproat, Zinn(II)-2-äthyl-caproat, Dibutylzinn(IV)-dilaurat oder Alkaliphenolat umgesetzt.

Geeignete Blockierungsmittel sind beispielsweise :

Monophenole wie Phenol, die Kresole, die Trimethylphenole, die tert. Butylphenole ; tertiäre Alkohole wie tert. Butanol, tert.-Amylalkohol, Dimethylphenylcarbonol ; leicht Enole bildende Verbindungen wie Acetessigester, Acetylaceton, Malonsäurederivate wie Malonsäurediester mit 1 bis 8 C-Atomen in den Alkoholresten ; sekundäre aromatische Amine wie N-Methylanilin, die N-Methyltoluidine, N-Phenyltoluidin, N-Phenylxylidin ; Imide wie Succinimid ; Lactame wie ε-Caprolactam, δ-Valerolactam ; Oxime wie Butanonoxim, Cyclohexanonoxim ; Mercaptane wie Methylmercaptan, Äthylmercaptan, Butylmercaptan, 2-Mercaptobenzthiazol, α-Naphthylmercaptan, Dodecylmercaptan, Triazole wie 1H-1,2,4-Triazol.

Zur Herstellung der Lackbindemittel werden gegebenenfalls blockiertes Polyisocyanat, polyfunktioneller Reaktionspartner, Katalysator und gegebenenfalls die üblichen Zusätze, wie z.B. Pigmente, Farbstoffe, Füllstoffe und Verlaufmittel miteinander auf einem üblichen Mischaggregat, wie z.B. auf einer Sandmühle entweder mit oder ohne Lösungs- und Verdünnungsmittel gut vermischt und homogenisiert.

Die Anstrich- und Überzugsmittel können in Lösung oder aus der Schmelze, oder in fester Form nach den üblichen Methoden wie z.B. Streichen, Rollen, Gießen, Spritzen, dem Wirbelsinterverfahren oder dem elektrostatischen Pulversprühverfahren auf den zu beschichtenden Gegenstand aufgebracht werden.

Die die erfindungsgemäßen Polyisocyanate enthaltenden Lacke ergeben Filme, die überraschend gut auf metallischem Untergrund haften, besonders lichtecht, wärmefarbstabil und sehr abriebfest sind und, sofern sie in lufttrocknenden Lacken verwendet werden, besonders rasch, selbst bei Temperaturen um 0 °C antrocknen. Darüber hinaus zeichnen sie sich durch große Härte, Elastizität, sehr gute Chemikalienbeständigkeit, hohen Glanz, ausgezeichnete Wetterbeständigkeit und gute Pigmentierbarkeit aus.

Die folgenden Beispiele erläutern die Erfindung. Alle Prozentangaben betreffen Gewichtsprozente.

In den nachfolgenden Beispielen 1 bis 9 und 11 wurden die folgenden gemäß US-PS 3 995 997 hergestellten Katalysatorlösungen verwendet :

### Katalysatorlösung I

2-Hydroxyäthyl-trimethylammoniumhydroxid, hergestellt durch Äthoxylierung von Trimethylamin in Wasser/Methanol (Volumenverhältnis = 1 : 1) bei ca. 40 °C und verdünnt auf eine 6 Gew.-% ige Lösung mit Dimethylformamid/Methanol (Volumenverhältnis = 4 : 1).

### Katalysatorlösung I-a

Wie Katalysatorlösung I, anstelle von Dimethylformamid/Methanol (4 : 1) hier 2-Äthylhexanol/Methanol (4 : 1).

### Katalysatorlösung II

2-Hydroxyäthyl-dodecyldimethylammoniumhydroxid, durch Äthoxylieren von Dodecyl-dimethylamin in $H_2O/CH_3OH$ bei ca. 40 °C, verdünnt auf eine 7,5 % ige Lösung mit Dimethylformamid/Methanol = 4 : 1.

### Katalysatorlösung III

Monoaddukt von Äthylenoxid an 1,4-Diazabicyclo-2,2,2-octan, durch Umsetzung von 1,4-Diazabicyclo-2,2,2-octan mit Äthylenoxid in $H_2O/CH_3OH$ bei ca. 40 °C und verdünnt mit Dimethylformamid/Methanol (4 : 1) auf eine 8 % ige Lösung.

Katalysatorlösung IV

2-Hydroxyäthyl-dimethyl-2,2'-dihydroxymethyl-butylammoniumhydroxid, aus Dimethyl-2,2'-dihydroxymethyl-butylamin Äthylenoxid, $H_2O$ und $CH_3OH$, verdünnt auf eine 20 % ige Lösung mit Methanol.

Beispiel 1

1332 g (6 Mol) IPDI werden in einem Dreihalskolben auf 80 °C erwärmt. Aus einem Tropftrichter werden innerhalb von 45 Minuten 15 ml der Katalysatorlösung I langsam und gleichmäßig zugetropft. Hierbei steigt die Temperatur auf ca. 88 °C (90 °C sollten nicht überschritten werden ; bei zu hoher Temperatur verläuft die Trimerisierung unspezifisch und führt zu höheren Viskositäten des Endproduktes). Es wird nach Beendigung des Zutropfens eine halbe Stunde nachgerührt, wobei die Temperatur auf 80 °C sinkt. Der NCO-Gehalt der Trimerisatlösung liegt hiernach bei 30,6 %. Im Hochvakuum wird gedünnschichtet und das Harz anschließend 75 % ig in Äthylglykolacetat gelöst.

Ausbeute (Harz) : 580 g (44 %)
Viskosität (Lösung) : 5107 m Pa-s (25 °C)
NCO-Gehalt (Lösg.) : 12,5 %
freies IPDI (Lösg.) : 0,18 %

Beispiel 2

1332 g (6 Mol) IPDI werden in einem Dreihalskolben vorgelegt und auf 50 °C erwärmt. Aus einem Tropftrichter läßt man 10 ml der Katalysatorlösung I-a zulaufen. Die Temperatur steigt innerhalb von ca. 30 Minuten auf 75 °C. Man erhöht hiernach auf 80 °C und läßt eine halbe Stunde bei dieser Temperatur nachrühren. Der NCO-Gehalt der Lösung beträgt hiernach 31,1 %. Durch Dünnschichtdestillation wird das überschüssige IPDI entfernt und das anfallende Harz 75 % ig in Äthylglykolacetat gelöst.

Ausbeute (Harz) : 485 g (39 %)
Viskosität (Lösg) : 5550 cp (25 °C)
NCO-Gehalt (Lösg) : 12,5 %
freies IPDI (Lösg) : 0,23 %

Beispiel 3

1332 g (6 Mol) IPDI werden bei Raumtemperatur mit 1 g Mannichbase der Formel

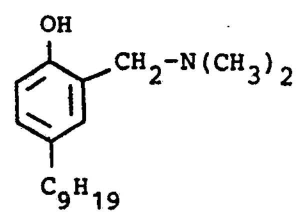

verrührt und auf 80 °C erwärmt. Aus einem Tropftrichter werden innerhalb von etwa 60 Minuten 15 ml der Katalysatorlösung I gleichmäßig eingetropft. Hierbei steigt die Temperatur langsam auf 85 °C. Diese erhöhte Temperatur wird durch das Zutropfen der Katalysatorlösung aufrechterhalten. Nach Beendigung der Trimerisierung werden 30 Minuten bei 80 °C nachgerührt. Hiernach weist die Trimerisatlösung einen NCO-Gehalt von 30,8 % auf. Zur Isolierung des Isocyanurats wird im Hochvakuum gedünnschichtet.

Ausbeute (Harz) : 568 g (42,6 %)
Die 75 % ige Lösung in Äthylglykolacetat hat eine Viskosität von 6576 mPa-s (25 °C) und einen NCO-Gehalt von 12,6 %
freies IPDI (Lösg.) : 0,36 %

Beispiel 4

Die gezielte Trimerisierung zu bestimmten NCO-Werten und den damit verbundenen Ausbeuten an Harz wird anhand der folgenden Beispiele gezeigt :

A) 1332 g IPDI werden auf 60 °C erwärmt. Aus einem Tropftrichter mit insgesamt 10 ml der Katalysatorlösung I werden portionsweise jeweils 2,5 ml zudosiert. Nach den ersten 2,5 ml steigt die Temperatur innerhalb von 15 Minuten auf 68 °C. Zu diesem Zeitpunkt läßt man weitere 2,5 ml Katalysatorlösung in die IPDI-Vorlage zulaufen. Die Temperatur steigt auf 78 °C. 15 Minuten nach der Zugabe beträgt der NCO-Gehalt der Lösung 34,9 %. Hiernach wird die Lösung 30 Minuten bei 80 °C

nachgerührt und anschließend gedünnschichtet (Ergebnisse siehe Tabelle).

B) In einem Parallelversuch mit 1332 g Isophorondiisocyanat werden 15 Minuten nach der zweiten Katalysatorzugabe (der NCO-Wert beträgt zu diesem Zeitpunkt 34,7 %) weitere 2,5 ml zudosiert. Die Temperatur steigt von 76 °C auf 80 °C. Nach 15 Minuten vom Zeitpunkt der Zugabe beträgt der NCO-Wert der Trimerisatlösung 32,7 %. Es wird eine halbe Stunde bei 80 °C nachgerührt und anschließend durch Dünnschichtdestillation aufgearbeitet (Ergebnisse siehe unten).

C) In einem weiteren Parallelversuch unter denselben Bedingungen werden weitere 2,5 ml Katalysatorlösung in die Trimerisatlösung zugegeben, die zu diesen Zeitpunkt nach insgesamt 3 Zudosierungen einen NCO-Gehalt von 32,5 % und eine Temperatur von 80 °C aufweist: 15 Minuten nach dieser Zudosierung beträgt der NCO-Gehalt 30,6 %, die Temperatur steigt nach der Zugabe von 80° auf 83 °C. Nach 30 minütigem Nachrühren wird wie in den vorigen Beispielen gedünnschichtet.

D) In einem vierten Versuch werden im Anschluß an die wie unter C) erfolgte Katalysierung weitere 1,5 ml Katalysatorlösung zugegeben. Die Temperatur steigt um 2° auf 84 °C an, der NCO-Wert sinkt von 30,5 auf 29 %. Nach 30 Minuten Nachrühren bei 80 °C wird durch Dünnschichtdestillation aufgearbeitet.

| Versuch | NCO-Gehalt n.d. Trimerisation | Ausbeute (Harz) | Viskosität (25 °C) (75 % ige Lösung) |
|---|---|---|---|
| A | 34,9 % | 250 g (18,8 %) | 2040 mPas |
| B | 32,7 % | 350 g (27 %) | 3520 mPas |
| C | 30,6 % | 545 g (41 %) | 5350 mPas |
| D | 29,0 % | 680 g (51 %) | 8730 mPas |

## Beispiel 5

50 kg IPDI werden in einem Rührkessel vorgelegt und auf 55 °C erwärmt. Aus einem Tropftrichter mit insgesamt 375 ml der Katalysatorlösung I läßt man 180 ml in das IPDI einlaufen. Die Temperatur steigt und erreicht innerhalb von 20 Minuten ihr erstes Maximum von 65 °C. Durch zwei weitere Zugaben von jeweils 75 ml Basenlösung wird die Trimerisierung weiter katalysiert, wobei nach der ersten Zugabe die Temperatur von 65 auf 72 °C und dann von 72 auf 80 °C steigt. Nach 15 minütigem Nachrühren beträgt der NCO-Gehalt 32,9 %, die Temperatur während des Nachrührens ist auf 75 °C gesunken. Zur Einstellung des angestrebten NCO-Wertes werden weitere 50 ml Katalysatorlösung zudosiert, die Temperatur steigt um 3 °C auf 78 °C. Es wird 1/2 Stunde nachgerührt und hiernach die Trimerisatlösung mit einem NCO-Gehalt von 31,1 % gedünnschichtet.

Ausbeute (Harz): 16,5 kg (35 %)
75 % ige Lösung in Äthylglykolacetat
Viskosität: 6500 mPas (25 °C)
NCO-Gehalt: 12,6 %
freies IPDI: 0,22 %

## Beispiel 6

1332 g (6 Mol) IPDI werden in einem Dreihalskolben vorgelegt und auf 60 °C erwärmt. Aus einem Tropftrichter mit insgesamt 20 ml der Katalysatorlösung II werden portionsweise und zwar zunächst 10 ml Katalysatorlösung zugegeben. Die Temperatur steigt innerhalb von 20 Minuten auf 67 °C an. Nachdem dieses Maximum erreicht ist, werden weitere 5 ml Lösung zugegeben, so daß die Temperatur auf 72 °C steigt. Hiernach beträgt der NCO-Gehalt der Trimerisatlösung 33,5 %. Mit weiteren 3 ml Katalysatorlösung wird ein Endwert von 30,3 % erreicht, wobei zuletzt die Temperatur auf 78 °C angestiegen ist. Bei 80 °C wird 1 Stunde nachgerührt und hiernach im Hochvakuum gedünnschichtet.

Ausbeute (Harz): 550 g (41,3 %)
75 % ige Lösung in Äthylglykolacetat:
Viskosität: 5750 mPas (25 °C)
NCO-Gehalt: 12,8 %
freies IPDI: 0,43 %

## Beispiel 7

In diesem Beispiel wird die Katalysatorlösung III verwendet. Aus einem Tropftrichter werden zunächst 8 ml dieser Lösung in einen Dreihalskolben mit 1332 g (6 Mol) IPDI von 60 °C gegeben. Die Temperatur steigt hiernach innerhalb von 20 Minuten auf 67 °C. Wie im vorigen Beispiel wird nun portionsweise mit jeweils 2,5 ml nachkatalysiert. Nach drei Zugaben, wobei die Temperatur am Ende

79 °C beträgt, ist ein NCO-Gehalt der Lösung von 31,5 % erreicht. Es wird eine halbe Stunde nachgerührt und anschließend im Hochvakuum gedünnschichtet. Das hierbei anfallende Harz wird 75 % ig in Äthylglykolacetat gelöst.

Ausbeute (Harz) : 450 g (33,8 %)
Viskosität (Lösung) : 4040 mPas (25 °C)
NCO-Gehalt (Lösung) : 12,9 %
freies IPDI (Lösung) : 0,3 %

Beispiel 8

Es wird in Analogie zu Beispiel 7 unter Verwendung der Katalysatorlösung IV gearbeitet. 1332 g (6 Mol) IPDI werden auf 80 °C erwärmt und insgesamt mit 8 ml Katalysatorlösung zu zwei Anteilen von je 3 ml und einmal 2 ml versetzt. Die Temperatur beträgt nach der dritten Zugabe 95 °C. Der NCO-Wert der Trimerisatlösung liegt hiernach bei 31,4 %. Es wird eine halbe Stunde bei 90 °C nachgerührt und anschließend im Hochvakuum gedünnschichtet.

Ausbeute (Harz) : 510 g (38 %)
75 % ige Lösung in Äthylglykolacetat :
Viskosität : 7500 mPas (25 °C)
NCO-Gehalt : 12,3 %
freies IPDI : 0,27 %

Beispiel 9

Ein auf 75 °C beheiztes Reaktionsgefäß wird über eine Dosierpumpe kontinuierlich mit 50 kg einer Mischung aus IPDI und 11 ml Katalysatorlösung I pro 1332 g IPDI beschickt. Die Förderleistung beträgt 11,1 l/h. Es stellt sich ein NCO-Gehalt der Reaktionslösung von 31 % ein. Nach 4,5 Stunden ist die gesamte Menge eindosiert. Es wird auf 80 °C erhitzt und 1/2 Stunde bei dieser Temperatur nachgerührt. Der End-NCO-Wert beträgt hiernach 30,5 %. Die Trimerisatlösung wird im Hochvakuum gedünnschichtet und das Harz hiernach 70 % ig in Xylol/Äthylglykolacetat 1 : 1 gelöst.

Ausbeute (Harz) : 18,2 kg (36 %)
Viskosität (Lösung) : 1370 mPas (20 °C)
NCO-Gehalt (Lösung) : 11,5 %
freies IPDI : 0,25 %

Beispiel 10

13,32 kg IPDI werden bei Raumtemperatur (23 °C) mit 65 ml Katalysatorlösung I gemischt. In einer Laborkaskade, bestehend aus drei 2 1-Rührgefäßen mit Überlauf, wird IPDI vorgelegt. In das erste auf 75 °C gehaltene Rührgefäß wird die obengenannte Abmischung mit einer Förderleistung von 3,25 l/h eingepumpt. Aus diesem Gefäß gelangt das Reaktionsprodukt zur Vervollständigung der Reaktion in den zweiten, auf 85 °C gehaltenen Reaktor. Die mittlere Verweilzeit in den beiden Kaskadenbehältern beträgt ca. 90 Minuten. Im dritten Reaktor wird der Katalysator bei 120 °C inaktiviert. Der NCO-Wert am letzten Ablauf beträgt 29 %. Das Rohprodukt wird in einen Dünnschichtverdampfer eingegeben.

Ausbeute (Harz) : 48 %
75 % ige Lösung in Äthylenglykolacetat :
Viskosität (20 °C) = 4937 mPas
NCO-Gehalt = 12,5 %
freies IPDI = 0,3 %

**Ansprüche**

1. Verfahren zur Herstellung von Isocyanuratgruppen aufweisenden Polyisocyanaten durch teilweise Trimerisierung der Isocyanatgruppen von 1-Isocyanato-3,3,5-trimethyl-5-isocyanatomethyl-cyclohexan unter Verwendung von die Trimerisierung von Isocyanatgruppen beschleunigenden Katalysatoren, dadurch gekennzeichnet, daß man als Katalysator mindestens eine Hydroxylgruppe aufweisende quaternäre Hydroxyalkylammonium-hydroxide der Formel

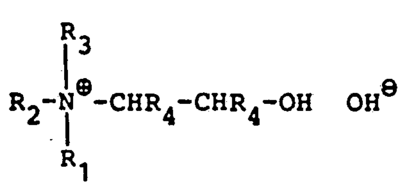

in welcher

$R_1$, $R_2$ und $R_3$ für gleiche oder verschiedene Reste Stehen und gegebenenfalls Hydroxyl-substituierte Alkylgruppen mit 1 bis 20 Kohlenstoffatomen, gegebenenfalls mit Hydroxylgruppen substituierte Cycloalkylreste mit 4 bis 15 Kohlenstoffatomen, gegebenenfalls mit Hydroxylgruppen substituierte Aralkylreste mit 7 bis 15 Kohlenstoffatomen oder gegebenenfalls mit Hydroxylgruppen substituierte Arylreste mit 6 bis 15 Kohlenstoffatomen bedeuten, wobei zwei der genannten Reste $R_1$, $R_2$ oder $R_3$ auch zusammen mit dem Stickstoffatom und gegebenenfalls zusammen mit einem Sauerstoff- oder einem weiteren Stickstoff-Heteroatom einen heterocyclischen Ring mit 4-6 Kohlenstoffatomen bilden können, oder wobei die Reste $R_1$, $R_2$ und $R_3$ jeweils für Äthylenreste stehen, die zusammen mit dem quartären Stickstoffatom und einem weiteren tertiären Stickstoffatom ein bicyclisches Triäthylen-diamin-Gerüst bilden,

$R_4$ für Wasserstoff und/oder eine Alkylgruppe mit 1 bis 12 Kohlenstoffatomen, eine Cycloalkylgruppe mit 5 bis 7 Kohlenstoffatomen, eine Aralkylgruppe mit 7 bis 10 Kohlenstoffatomen, eine Arylgruppe mit 6 bis 10 Kohlenstoffatomen oder eine $R_5$-O-$(CH_2)_n$-Gruppe darstellt, in welcher $R_5$ für Wasserstoff, einen Alkylrest mit 1 bis 12 Kohlenstoffatomen, einen Cycloalkylrest mit 4 bis 10 Kohlenstoffatomen, einen Aralkylrest mit 7 bis 10 Kohlenstoffatomen oder einen Arylrest mit 6 bis 10 Kohlenstoffatomen steht und n eine ganze Zahl von 1 bis 6 bedeutet

verwendet.

2. Verfahren gemäß Anspruch 1, dadurch gekennzeichnet, daß man den Katalysator in einer Menge von 0,01 bis 1 Gew.-% bezogen auf zu trimerisierendes Diisocyanat verwendet.

3. Verfahren gemäß Anspruch 1 und 2, dadurch gekennzeichnet, daß man den Katalysator dem auf 40 bis 110 °C vorerhitzten Diisocyanat zumischt.

4. Verwendung der gemäß Anspruch 1 bis 3 erhältlichen Verfahrensprodukte gegebenenfalls in mit Blockierungsmitteln für Isocyanatgruppen blockierter Form als Isocyanatkomponente in Polyurethanlacken.

**Claims**

1. Process for the preparation of polyisocyanates containing isocyanurate groups by the partial trimerisation of the isocyanate groups of 1-isocyanato-3,3,5-trimethyl-5-isocyanatomethyl-cyclohexane using catalysts which accelerate the trimerisation of isocyanate groups, characterised in that the catalysts used are quarternary hydroxy alkyl ammonium hydroxides, containing at least one hydroxyl group, of the formula

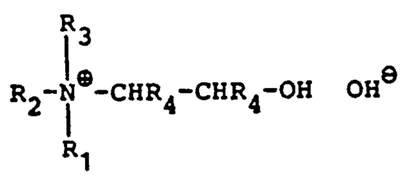

in which

$R_1$, $R_2$ and $R_3$ represent identical or different groups and denote alkyl groups with 1 to 20 carbon atoms, which may be substituted with hydroxyl, cycloalkyl groups with 4 to 15 carbon atoms, which may be substituted with hydroxyl groups, aralkyl groups with 7 to 15 carbon atoms which may be substituted with hydroxyl groups or aryl groups with 6 to 15 carbon atoms which may be substituted with hydroxyl groups, or two of the aforesaid groups $R_1$, $R_2$ and $R_3$ may also combine with the nitrogen atom and optionally with an oxygen atom or another nitrogen hetero atom to form a heterocyclic ring containing 4 to 6 carbon atoms, or the groups $R_1$, $R_2$ and $R_3$ may each represent ethylene groups which form a bicyclic triethylene diamine structure together with the quarternary nitrogen atom and another tertiary nitrogen atom ;

$R_4$ represents hydrogen and/or an alkyl group with 1 to 12 carbon atoms, a cycloalkyl group with 5 to 7 carbon atoms, an aralkyl group with 7 to 10 carbon atoms, an aryl group with 6 to 10 carbon atoms or a group of the formula $R_5$-O-$(CH_2)_n$- in which $R_5$ represents hydrogen, an alkyl group with 1 to 12 carbon atoms, a cycloalkyl group with 4 to 10 carbon atoms, an aralkyl group with 7 to 10 carbon atoms or an aryl group with 6 to 10 carbon atoms and n is an integer of from 1 to 6.

2. Process according to Claim 1, characterised in that the catalyst is used in a quantity of from 0.01 to 1 % by weight, based on the diisocyanate to be trimerised.

3. Process according to Claims 1 and 2, characterised in that the catalyst is added to the diisocyanate which has been preheated to 40 to 110 °C.

4. Use of the products of the process obtainable according to Claims 1 to 3, optionally in the form of products which have been blocked with blocking agents for isocyanate groups, as isocyanate components in polyurethane lacquers.

**Revendications**

1. Procédé de production de polyisocyanates porteurs de groupes isocyanurate par trimérisation partielle des groupes isocyanate du 1-isocyanato-3,3,5-triméthyl-5-isocyanatométhyl-cyclohexane en présence de catalyseurs accélérant la trimérisation des groupes isocyanate, caractérisé en ce qu'on utilise comme catalyseur des hydroxydes d'hydroxy-alkylammonium quaternaire, porteurs d'au moins un groupe hydroxyle, de formule :

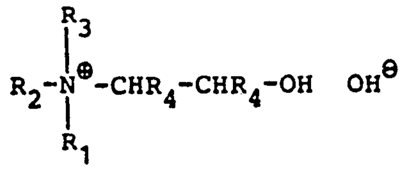

dans laquelle

$R_1$, $R_2$ et $R_3$ sont des restes égaux ou différents et représentent des groupes alkyle ayant 1 à 20 atomes de carbone éventuellement substitués avec le groupe hydroxyle, des restes cycloalkyle ayant 4 à 15 atomes de carbone éventuellement substitués avec des groupes hydroxyle, des restes aralkyle ayant 7 à 15 atomes de carbone éventuellement substitués avec des groupes hydroxyle ou des restes aryle ayant 6 à 15 atomes de carbone éventuellement substitués avec des groupes hydroxyle, deux des restes $R_1$, $R_2$ ou $R_3$ mentionnés pouvant également former, avec l'atome d'azote et, le cas échéant, avec un hétéro-atome d'oxygène ou un autre hétéro-atome d'azote, un noyau hétérocyclique ayant 4 à 6 atomes de carbone, ou bien les restes $R_1$, $R_2$ et $R_3$ représentent chacun un reste éthylène et forment, avec l'atome d'azote quaternaire et un autre atome d'azote tertiaire, un squelette triéthylène-diamine bicyclique,

$R_4$ représente de l'hydrogène et/ou un groupe alkyle ayant 1 à 12 atomes de carbone, un groupe cycloalkyle ayant 5 à 7 atomes de carbone, un groupe aralkyle ayant 7 à 10 atomes de carbone, un groupe aryle ayant 6 à 10 atomes de carbone ou un groupe $R_5$-O-$(CH_2)_n$- dans lequel $R_5$ est de l'hydrogène, un reste alkyle ayant 1 à 12 atomes de carbone, un reste cycloalkyle ayant 4 à 10 atomes de carbone, un reste aralkyle ayant 7 à 10 atomes de carbone ou un reste aryle ayant 6 à 10 atomes de carbone et *n* est un nombre entier de 1 à 6.

2. Procédé suivant la revendication 1, caractérisé en ce qu'on utilise le catalyseur en une quantité de 0,01 à 1 % en poids par rapport au diisocyanate à trimériser.

3. Procédé suivant l'une des revendications 1 et 2, caractérisé en ce qu'on adjoint le catalyseur au diisocyanate préalablement chauffé à une température de 40 à 110 °C.

4. Utilisation des produits pouvant être obtenus par le procédé suivant les revendications 1 à 3, le cas échéant sous une forme bloquée par des agents de blocage des groupes isocyanate, comme composant isocyanate dans des vernis de polyuréthanne.